(19) **Europäisches Patentamt / European Patent Office / Office européen des brevets**

(11) **EP 4 285 860 B1**

(12) **EUROPEAN PATENT SPECIFICATION**

(45) Date of publication and mention
of the grant of the patent:
**29.04.2026 Bulletin 2026/18**

(21) Application number: **21954348.5**

(22) Date of filing: **30.11.2021**

(51) International Patent Classification (IPC):
**A61C 8/00** *(2006.01)* **A61L 27/06** *(2006.01)*
**A61L 27/10** *(2006.01)* **B23K 26/352** *(2014.01)*

(52) Cooperative Patent Classification (CPC):
**A61C 8/0012; A61C 8/0013; A61C 8/0022;**
A61C 2008/0046; A61F 2/30767; A61F 2002/30332;
A61F 2002/3084; A61F 2002/3085;
A61F 2002/30925; A61F 2002/3093

(86) International application number:
**PCT/KR2021/017891**

(87) International publication number:
**WO 2023/022302 (23.02.2023 Gazette 2023/08)**

(54) **SURFACE-TREATED IMPLANT STRUCTURE**

OBERFLÄCHENBEHANDELTE IMPLANTATSTRUKTUR

STRUCTURE D'IMPLANT TRAITÉE EN SURFACE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2021 KR 20210110309**

(43) Date of publication of application:
**06.12.2023 Bulletin 2023/49**

(73) Proprietor: **B2Lab Co., Ltd.
Seoul 05836 (KR)**

(72) Inventors:
• **JEONG, Bo Su
Seoul 04318 (KR)**

• **LEE, Byung Hak
Seoul 06764 (KR)**

(74) Representative: **ZBM Patents - Zea, Barlocci &
Markvardsen
Rambla de Catalunya, 123
08008 Barcelona (ES)**

(56) References cited:
WO-A1-2015/033680    WO-A1-2020/211746
IT-A1- UB20 155 412    JP-A- 2009 045 246
KR-A- 20110 091 257    KR-A- 20120 004 204
KR-A- 20190 052 689    KR-B1- 102 173 456
US-A1- 2020 345 465

**Description**

TECHNICAL FIELD

One or more embodiments relate to a surface-treated implant structure.

BACKGROUND ART

Implants are used for treatments for restoring the function of bones by using materials that are harmless to the human body. For example, existing implants are being used for indications related to bone tissues in dentistry, orthopedics, etc. Among these, dental implants are used to restore the function of teeth by fixing artificial teeth after implanting a fixture made of a material that does not react against the human body to replace the lost tooth root.

In the case of general prostheses or dentures, the surrounding teeth and bones are damaged over time, but implants can prevent damage to the surrounding dental tissues and can be used stably because there is no secondary cause of caries. In an embodiment, since the implant has the same structure as a natural tooth, there is no pain or foreign body feeling in the gums, and it can be used semi-permanently if well managed.

Materials used for such implants can be categorized as metals (for example, titanium (Ti), Co-Cr, SS, Gold, etc.), ceramics (for example, zirconia, alumina, etc.), and polymers (for example, PMMA). Currently, the most used material in Korea is metal or ceramic. Among metal materials, in the cases of titanium metal or titanium alloy, osseointegration takes a long time and an oxide film is formed, although stability is secured compared to other metals when implanted in the human body.

In an embodiment, the existing implants were used with coatings that prevent various biofilms on the implant surface depending on the surrounding environment applied for antibacterial purposes. For example, in the case of dental implants used for prosthetics and orthodontics, which are dental treatments, they were used with coatings to prevent biofilms depending on the oral environment. However, when a coating that prevents biofilms is applied to the surface of dental implant materials, inflammation (periimplantitis, etc.) and secondary caries may be induced, thereby increasing medical expenses, deterioration of oral health, and deterioration of national health insurance finances. Therefore, it is necessary to secure a next-generation technology that can replace the technology of applying a coating to prevent biofilms on the surface of the implant material (Korean Patent No. 2173456).

WO2015033680 disloses a titanium or titanium-alloy fixture embedded in bone for osseointegration with its external surface having laser-scanned grooves with 10-100 $\mu$m width and 1-30 $\mu$m depth, and a 3D roughness Sa of 1-5 $\mu$m. The surface is additionally roughened by acid etching.

DESCRIPTION OF EMBODIMENTS

TECHNICAL PROBLEM

One aspect is to provide an implant structure including a fixture that acts as an artificial tooth root, wherein the fixture has the surface including nano-protrusions having a certain level of height and an aspect ratio on the surface thereof.

One aspect is to provide an implant structure including a fixture that acts as an artificial tooth root, wherein the fixture is made by using a femtosecond laser, and has an outer peripheral surface having a structure with the level higher or lower than the unprocessed surface thereof.

SOLUTION TO PROBLEM

The invention is an implant structure as defined in claim 1. Further details of the invention are defined in the appended claims.

One aspect provides an implant structure including a fixture that acts as an artificial tooth root, wherein the fixture includes a cylindrical bone contact portion 111 having a first outer peripheral surface in which a thread is formed and a gingival contact portion 112 having a second outer peripheral surface, disposed above the second outer peripheral surface, wherein the first outer peripheral surface or the second outer peripheral surface includes a plurality of nano-protrusions having a width of 10 nm to 1000 nm and an aspect ratio of 1000:1 to 1:50.

One aspect provides an implant structure including a fixture that acts as an artificial tooth root, wherein the fixture includes a cylindrical bone contact portion 111 having a first outer peripheral surface in which a thread is formed and a gingival contact portion 112 having a second outer peripheral surface and disposed above the second outer peripheral surface, wherein the first outer peripheral surface or the second outer peripheral surface includes nano-protrusions having a width of 50 nm to 200 nm and an aspect ratio of 50:1 to 1:50.

In an embodiment, the fixture 110 constituting the implant structure may be provided in a form as being integrated with or separated from an abutment 140, and the fixture 110 may include the bone contact portion 111 including the outer peripheral surface in which a thread is formed and the gingival contact portion 112.

In an embodiment, the nano-protrusions may be arranged such that the surface of the implant structure in which the nano-protrusions are not formed is located between adjacent nano-protrusions.

The term "width" used herein refers to the distance between the centers of the ends of adjacent nano-protrusions included in the surface of the implant structure. The width may be in a range of about 1 nm to about 2000 nm, about 1 nm to about 1000 nm, about 5 nm to about 1000 nm, about 10 nm to about 1000 nm, about 100 nm to about 1000 nm, about 10 nm to about 500 nm, or about 10 nm to about 100 nm. The nano-protrusions having a width of about 1 nm to 2000 nm may impart unique surface properties and functionality to the first outer peripheral surface or the second outer peripheral surface of the implant structure, wherein the functionality may be an antibacterial effect or an osseointegration promoting effect.

In an embodiment, the width of the adjacent nano-protrusions and the horizontal length or vertical length of the nano-protrusions may be in the range of 1:1 to 100:1, 1:1 to 50:1, or 1:1 to 10:1.

The term "aspect ratio" used herein refers to the ratio of the width to length of the nano-protrusions included in the surface of the implant structure. The aspect ratio of the width to the length may be in the range of 1000:1 to 1:50, 500:1 to 1:50, 200:1 to 1:50, 100:1 to 1:50, 50:1 to 1:50, 40:1 to 1:40, 30:1 to 1:30, or 20:1 to 1:20. The nano-protrusions having the aspect ratio of 1000:1 to 1:50 may impart unique surface properties and functionality to the first outer peripheral surface or the second outer peripheral surface of the implant structure, wherein the functionality may be an antibacterial effect or an osseointegration promoting effect. The first outer peripheral surface or the second outer peripheral surface may be used interchangeably with the surface of the implant structure.

In an embodiment, when the surface of the implant structure has a plurality of nano-protrusions having the aspect ratio of 1:1 to 1:50, 1:1 to 1:45, 1:1 to 1:40, 1:1 to 1:35, 1:1 to 1:30, 1:1 to 1:25, 1:1 to 1:20, 1:1 to 1:15, 1:1 to 1:10, 1:10 to 1:50, 1:10 to 1:45, 1: An aspect ratio of 10 to 1:40, 1:10 to 1:35, 1:10 to 1:30, 1:10 to 1:25, 1:10 to 1:20, or 1:10 to 1:15, the surface of the implant structure may exhibit an antibacterial effect of killing germs such as bacteria.

In an embodiment, when the surface of the implant structure has a plurality of nano-protrusions having an aspect ratio of 1000:1 to 1:1, 100:1 to 1:1, 50:1 to 1:1, 40:1 to 1:1, 30:1 to 1:1, 20:1 to 1:1, 10:1 to 1:1, 1000:1 to 10:1, 100:1 to 10:1, 50:1 to 10:1, 40:1 to 10:1, 30:1 to 10:1, or 20:1 to 10:1, the surface of the implant structure may exhibit an osseointegration promoting effect.

In an embodiment, depending on the processing type from among the surface treatment conditions, in the case of the linear type, the implant structure may have a plurality of nano-protrusions having the aspect ratio of 1000:1 to 10:1, and in the case of the grid type, the implant structure may have a plurality of nano-protrusions having the aspect ratio of 1:1 to 1:50.

In an embodiment, the nano-protrusion shape of the second outer peripheral surface promotes the death of bacteria present on the implant surface, and the nano-protrusions may have a width of about 10 nm to about 100 nm and an aspect ratio of 1:50 to 1:1, or a width of about 100 nm to about 1000 nm and an aspect ratio of 1:1 to 1:50. In an embodiment, the nano-protrusion shape of the second outer peripheral surface may be provided with a height of about 100 nm to about 200 nm and an aspect ratio of 1:1 to 1:50.

In an embodiment, the nano-protrusions may be provided with a blunt end shape.

The term "blunt" used herein refers to a case in which a value obtained by multiplying the "horizontal ratio of the nano-protrusions" and the "curvature of the ends of the nano-protrusions" is from -2 to +2. For example, the curvature may be from -2 to +2, from -1 to +1, or from -0.5 to +0.5, but is not limited thereto.

In an embodiment, when the value obtained by multiplying the "horizontal length of nano-protrusions" and the "curvature of ends of the nano-protrusions" of the nano-protrusions is +2, the ends of the nano-protrusions may have a hemispherical shape.

In an embodiment, when the ends of the nano-protrusions do not form a curvature or is less than a certain level of curvature, the ends of the nano-protrusions may have a pointed shape.

In an embodiment, an arithmetic mean roughness (Ra) value of the second outer peripheral surface may be in a range of about 1.0 $\mu$m to about 5.0 $\mu$m. The arithmetic mean roughness (Ra) value may be, for example, about 1.0 $\mu$m to about 4.00 $\mu$m, about 1.0 $\mu$m to about 3.0 $\mu$m, about 1.00 $\mu$m to about 2.0 $\mu$m, about 2.0 $\mu$m to about 5.0 $\mu$m, about 2.0 $\mu$m to about 4.0 $\mu$m, about 2.0 $\mu$m to about 3.0 $\mu$m, about 3.0 $\mu$m to about 5.0 $\mu$m, or about 3.0 $\mu$m to about 4.0 $\mu$m. The arithmetic mean roughness of the surface as described above may contribute to promoting the killing of bacteria present on the implant surface.

The term "arithmetic mean roughness (Ra)" used herein refers to an arithmetic mean height of a roughness curve, and the parameter value may be defined according to ISO 25178-2:2012 standards.

According to the invention, the nano-protrusion shape of the first outer peripheral surface is to promote osseointegration with the implant, and is provided with a width of 10 nm to 1000 nm and an aspect ratio of 1000:1 to 1:1. For example, the nano-protrusions shape of the first outer peripheral surface may have a width of 10 nm to 1000 nm and an aspect ratio of 100:1 to 1:1. In an embodiment, the nano-protrusions shape of the first outer peripheral surface may have a height of 50 nm to 200 nm and an aspect ratio of 1000:1 to 1:1.

In an embodiment, an arithmetic mean roughness (Ra) value of the first outer peripheral surface may be in a range of 0.5 μm to 3.0 μm. The arithmetic mean roughness (Ra) value may be, for example, 0.5 μm to 2.0 μm, 0.5 μm to 1.0 μm, 1.0 μm to 3.0 μm, 1.0 μm to 2.5 μm, 1.0 μm to 2.0 μm, or 2.0 μm to 3.0 μm. The arithmetic mean roughness of the surface as described above may contribute to promoting osseointegration with the implant.

The aspect ratio of the nano-protrusions included in the first outer peripheral surface may be different from the aspect ratio of the nano-protrusions included in the second outer peripheral surface. The bone contact portion including the first outer peripheral surface includes nano-protrusions having a surface property for promoting osseointegration, that is, an aspect ratio range of 1000:1 to 1:1, and the gingival contact portion having the second outer peripheral surface includes nano-protrusions having a surface property for exhibiting an antibacterial effect, that is, an aspect ratio of 1:1 to 1:50.

In an embodiment, the implant structure may be surface-treated with a femtosecond laser. The surface treatment may be irradiating of a femtosecond laser beam having a laser intensity of 1 W to 5 W on the surface of the first outer peripheral surface or the second outer peripheral surface in a linear-type and grid-type one or more times.

Another aspect provides an implant structure including a fixture that acts as an artificial tooth root, wherein the fixture includes a cylindrical bone contact portion 111 including an outer peripheral surface having a thread formed thereon, and the outer peripheral surface includes a bone formation facilitation region 113 including a plurality of nano-protrusions having a width of 10 nm to 1000 nm and an aspect ratio of 1000:1 to 1:1, and an antimicrobial activity facilitation region 114 located on the bone formation facilitation region and including a plurality of nano-protrusions having a width of 10 nm to 1000 nm and an aspect ratio of 1:1 to 1:50.

In an embodiment, the fixture 110 constituting the implant structure may be provided in a form as being integrated with or separated from an abutment 140, and the fixture 110 may consist of the bone contact portion 111 including the outer peripheral surface in which a thread is formed.

In an embodiment, the bone contact portion 111 may include the bone formation facilitation region 113 and the antimicrobial activity facilitation region 114, or may consist of the bone formation facilitation region 113 and the antimicrobial activity facilitation region 114, and the antimicrobial activity facilitation region 114 may be located above the bone formation facilitation region 113 or on the end of the bone formation facilitation region 113.

In an embodiment, the surface of the bone formation facilitation region 113 may have a plurality of nano-protrusions having the aspect ratio of 1:1 to 1:50, 1:1 to 1:45, 1:1 to 1:40, 1:1 to 1:35, 1:1 to 1:30, 1:1 to 1:25, 1:1 to 1:20, 1:1 to 1:15, 1:1 to 1:10, 1:10 to 1:50, 1:10 to 1:45, 1:10 to 1:40, 1:10 to 1:35, 1:10 to 1:30, 1:10 to 1:25, 1:10 to 1:20, or 1:10 to 1:15.

In an embodiment, the surface of the antimicrobial activity facilitation region 114 may have a plurality of nano-protrusions having an aspect ratio of 1000:1 to 1:1, 100:1 to 1:1, 50:1 to 1:1, 40:1 to 1:1, 30:1 to 1:1, 20:1 to 1:1, 10:1 to 1:1, 1000:1 to 10:1, 100:1 to 10:1, 50:1 to 10:1, 40:1 to 10:1, 30:1 to 10:1, or 20:1 to 10:1.

In an embodiment, the implant structure may have been subjected to a surface treatment using a femtosecond laser, and depending on the processing form among the surface treatment conditions, in the case of a linear type, an antibacterial activity facilitation region having a plurality of nano-protrusions having an aspect ratio of 1000:1 to 10:1 may be provided, and in the case of a grid type, a bone formation facilitation region having a plurality of nano-protrusions having an aspect ratio of 1:1 to 1:50 may be provided.

In an embodiment, the nano-protrusions of the antimicrobial activity facilitation region 114 may have blunt ends.

In one embodiment, the antimicrobial activity facilitation region 114 may be formed in the range of 0.1 mm to 3.0 mm in the direction extending from the top of the bone contact portion 111 to the bone formation promoting region 113. In an embodiment, the length of the antimicrobial activity facilitation region 114 may be from 0.1 mm to 3.0 mm, for example, 0.1 mm, 0.5 mm, 1.0 mm, 1.5 mm, 2.0 mm, 2.5 mm, or 3.0 mm, in the direction extending from the top end of the bone contact portion to the bone formation promoting region 113. Here, the top end of the bone contact portion 111 may refer to an area thereof adjacent to the cross-section of the fixture exposed to the outside after being inserted into the alveolar bone, and the length may be used interchangeably with the height in the vertical axis of the fixture. In an embodiment, the antimicrobial activity facilitation region 114 may be 1% to 50%, for example, 1% to 40%, 1% to 30%, 1% to 20%, 1% to 10%, 1% to 5%, 10% to 50%, 10% to 40%, 10% to 30%, or 10% to 20%, based on the total length of the bone contact portion 111.

Regarding the detailed description of the fixture having the bone formation facilitation region and the antibacterial activity facilitation region, and the implant structure including the same, the same or corresponding technical configuration as the implant structure having the bone contact portion and the gingival contact portion may be used or applied in parallel herein.

In an embodiment, the implant structure may be prepared using a biocompatible material. The biocompatible material is not limited to a metal, ceramic, or resin, as long as the biocompatible material is a suitable material. For example, the biocompatible metal material may be copper, titanium, titanium alloy, cobalt chromium alloy, etc., the biocompatible ceramic material may be alumina (aluminum oxide), yttrium oxide, hafnium oxide, silicon oxide, magnesium oxide, cerium oxide, and the biocompatible resin material may be silicone, nylon, POM, or a composite material.

In an embodiment, titanium or titanium alloy may be included in 50% or more and 100% or less in the volume ratio with respect to the implant. For example, titanium may be included in 50% or more and 100% or less, 60% or more and 99% or less, 70% or more and 95% or less, 80% or more and 95% or less, or 90% or more and 95% or less in the volume ratio with respect to the implant.

In an embodiment, the implant structure may be subjected to a laser surface treatment, a blasting surface treatment, or an etching surface treatment. For example, the implant structure may be provided by simultaneously performing a blasting process, in which surface roughness is increased by spraying fine sand particles on the first outer peripheral surface or the second outer peripheral surface, and an etching process, in which surface roughness is increased by immersing in an etchant to corrode the surface. In an embodiment, the implant structure may be subjected to the surface treatment using a laser. For example, the laser may be a femtosecond laser.

The blasting surface treatment used herein refers to a process in which the surface roughness is increased by spraying fine particles on the surface of the implant structure. For example, the surface roughness can be increased by spraying fine sand particles or the like on the first outer peripheral surface and the second outer peripheral surface. Although sand is usually used, when a hard material such as boron carbide is used, a significantly improved macroscopic roughness may be obtained. For example, $Al_2O_3$ particles having an average diameter of 250 $\mu$m to 500 $\mu$m may be used.

The etching surface treatment used herein may be performed by immersing the bone contact portion 111 and the gingival contact portion 112 in an etching solution to corrode the surface, and due to the corrosion, the surface roughness of the implant structure may be increased. For example, the etching may be performed by reducing an acid such as hydrofluoric acid (HF), or a mixture of hydrochloric acid (HCL) and sulfuric acid ($H_2SO_4$). However, the etching solution is not limited thereto, and may contain at least one compound selected from phosphoric acid, nitric acid, ammonium fluoride, hydrogen peroxide, and hydrobromic acid.

The etching time varies depending on the etching solution used, and may generally be from about 10 seconds to about 120 minutes. For etching, at least a portion of the implant structure may be immersed in an etching solution for about 1 minute to 60 minutes, from about 20 minutes to 40 minutes, or about 30 minutes. However, the present disclosure is not limited thereto, and only the bone contact portion 111 or the gingival contact portion 112 may be immersed in the etching solution.

After the etching surface treatment, an implant structure 100 may be additionally cleaned by a cleaning solution.

In the present specification, the blasting process may provide a relatively large roughness to the surface of the first outer peripheral surface or the second outer peripheral surface, and the etching process may provide a slightly lower roughness than the blasting process. However, the present disclosure is not limited thereto, and the surface of the first outer peripheral surface or the second outer peripheral surface may be treated only by etching.

Another aspect provides an implant structure having a fixture that acts as an artificial tooth root, wherein the fixture includes a cylindrical bone contact portion having a first outer peripheral surface in which a thread is formed and a gingival contact portion disposed on the bone contact portion and including a second outer peripheral surface, wherein the first outer peripheral surface or the second outer peripheral surface is surface treated by using a femtosecond laser, and (i) the height of the to-be-processed surface of the first outer peripheral surface or the second outer peripheral surface is 0.001 mm to 10 mm greater than the height of the surface which has not been subjected to the laser processing, or (ii) the depth of the to-be-processed surface of the first outer peripheral surface or the second outer peripheral surface is 0.001 mm to 10 mm smaller than the depth of the surface which has not been subjected to the laser processing.

In an embodiment, the height of the to-be-processed surface of the first outer peripheral surface or the second outer peripheral surface may be 0.001 mm to 10 mm, 0.01 mm to 10 mm, or 0.1 mm to 10 mm greater than the height of the surface which has not been subjected to the laser processing

In an embodiment, the depth of the to-be-processed surface of the first outer peripheral surface or the second outer peripheral surface may be 0.001 mm to 10 mm, 0.01 mm to 10 mm, or 0.1 mm to 10 mm smaller than the depth of the surface which has not been subjected to the laser processing.

When the height of the first outer peripheral surface or the second outer peripheral surface is greater or smaller than that of the surface that is not processed, unique surface properties and functionality can be imparted to the first outer peripheral surface or the second outer peripheral surface of the implant structure. The functionality herein may be an antibacterial effect or an osseointegration facilitation effect.

In an embodiment, the first outer peripheral surface has a structure that facilitates osseointegration, wherein (i) the height of the to-be-processed surface of the first outer peripheral surface is 0.001 mm to 10 mm greater than that of the surface which has not been subjected to the laser processing or (ii) the depth of the to-be-processed surface of the first outer peripheral surface is 0.001 mm to 10 mm smaller than that of the surface which has not been subjected to the laser processing.

In an embodiment, the height of the to-be-processed surface of the first outer peripheral surface may be 0.001 mm to 10 mm, 0.01 mm to 10 mm, or 0.1 mm to 10 mm greater than the height of the surface which has not been subjected to the laser processing.

In an embodiment, the depth of the to-be-processed surface of the first outer peripheral surface may be 0.001 mm to 10 mm, 0.01 mm to 10 mm, or 0.1 mm to 10 mm smaller than the depth of the surface which has not been subjected to the laser processing.

Another aspect provides a surface treatment method for an implant structure, the surface treatment method including: setting processing conditions of a laser beam; providing a laser beam according to a set condition; and treating the surface of the implant structure.

In an embodiment, in the setting of the processing conditions of the laser beam, a laser processing speed is set to be from 0.1 mm/s to 2500 mm/s, a laser intensity is set to be from 0.1 W to 100 W, and a laser intensity is set to be from 0.1 J/cm$^2$ to 100 J/cm$^2$.

The term "laser intensity" used herein refers to energy per unit area and a final result value calculated by comprehensively considering the average power, repetition rate, irradiation area, etc. during actual laser processing.

In an embodiment, in the treating of the surface, the surface of the implant structure may be treated by irradiating the laser beam so as to have a uniform surface treatment area within the depth-of-focus area corresponding to the direction in which the laser beam travels.

In an embodiment, in the treating of the surface, the peak intensity at the focus of the laser may be set to be four times the critical intensity of the surface treatment of the implant. In this case, the peak intensity to have a uniform surface treatment area may be calculated through Equation 1 below.

[Equation 1]

$$F_{peak,\,in\text{-}focus} \cong 4 \cdot F_{LIAT}$$

Here, $F_{peak,\,in\text{-}focus}$ is the $_{peak}$ fluence at the focus of the laser beam, and $F_{LIAT}$ is the critical intensity of the surface treatment of the three-dimensional object. The fluence is a unit of laser intensity in J/cm$^2$.

In an embodiment, the treating of the surface may be performed in such a manner that the surface of the implant structure is located within a depth of focus (DOF) corresponding to the direction in which the laser travels. In this case, the depth-of-focus area having a constant surface treatment area may be calculated through Equation 2 below.

[Equation 2]

$$DOF = 1.57 \frac{\lambda (M^2)^2}{NA^2}$$

Here, NA is the numerical aperture of the optical system, $\lambda$ is the center wavelength of the laser beam, and $M^2$ is the quality of the laser beam.

Using the above-described conditions, the laser beam may be treated to have a uniform surface treatment area within the depth-of-focus area corresponding to the direction in which the laser beam travels. Through such laser beam processing, the entire object may be uniformly surface treated by adjusting only the focal position of the laser beam, without the need to adjust the focal length of the laser beam according to the shape of the object.

Meanwhile, in the present disclosure, the surface treatment area generated by the unit laser pulse has a circular shape, but the present disclosure is not limited thereto. Accordingly, it would be obvious to those skilled in the art that the laser surface treatment area may be formed in a different shape according to the pattern of the laser beam.

In an embodiment, the depth-of-focus (DOF) area generated corresponding to the direction in which the laser beam travels may be provided on a portion to be subjected to the surface treatment, that is, the surface of an object 270 (for example, the first outer peripheral surface or the second outer peripheral surface). This is because uniform surface treatment can be obtained without the need to adjust the focal length (that is, the distance between the beam focusing unit and the focal point) only when the portion to be subjected to the surface treatment is located within the DOF of the laser beam. Accordingly, a laser surface treatment apparatus 200 which allows the surface of the object 270 to be within a DOF area corresponding to the direction in which the laser beam travels by adjusting the distance between a beam focusing unit 240 and the object 270 or by adjusting the optical characteristics of the beam focusing unit 240, may be implemented.

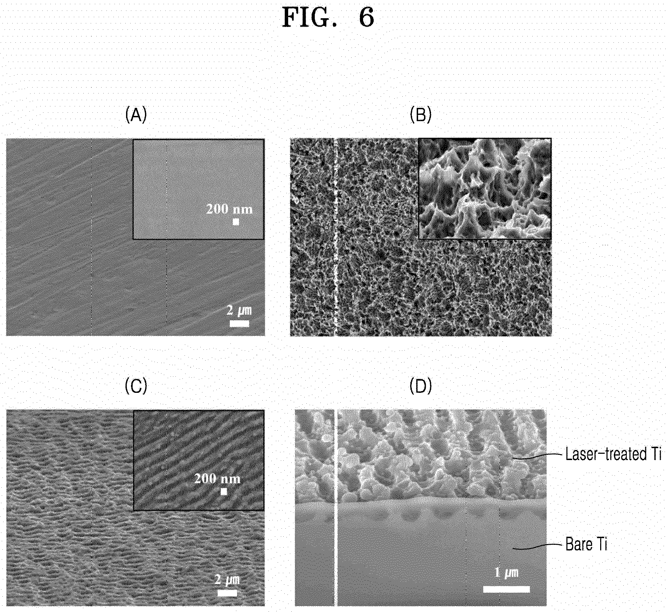

In an embodiment, when the surface of the implant structure is processed by a femtosecond laser, a linear type or grid type may be selected. For example, during the linear treatment, the line formed by the nano-protrusions on the surface of the implant structure (for example, arrangement in a line or mountain range, which is identifiable in the 200 nm enlarged view of FIG. 6C) or the bone forming the width of the nano-protrusions, can be processed clearly depending on the number of times. In an embodiment, during the grid-type treatment, the aspect ratio of the nano-protrusions on the surface of the implant structure may be increased.

In an embodiment, in the case of the surface treatment using a femtosecond laser, even when the portion of which surface is to be treated is away from the laser focal region by a certain distance, the surface treatment can be performed. For example, even when the distance is 8 mm to 12 mm (4 to 6 mm below or 4 to 6 mm above the portion that is not surface treated), the surface treatment can be performed. Since the surface treatment can be performed as described above, it is possible to surface-treat the threads of the implant fixture having a height difference of less than 10 mm at a time.

In the above-described embodiment, the dental implant and the surface treatment therefor have been described, but the present disclosure is not limited thereto.

Another aspect may provide an implant including, on the surface thereof, nano-protrusions having a width of 10 nm to 1000 nm and an aspect ratio of 1000:1 to 1:50.

Another aspect may provide an implant including, on the surface thereof, nano-protrusions having a width of 50 nm to 200 nm and an aspect ratio of 1000:1 to 1:50.

The implant may include a bone tissue replacement implant, a granular bulk filler to be included in a composition for filling a bone tissue defect, a bone screw, an implant for bone fusion, a fixation pin implant or a pin for implant, an implant for skull reconstruction, orthopedic surgery implants, percutaneous osseointegration implants, and dental implants.

For example, orthopedic implants may include hip joint implants, knee joint implants, shoulder joint implants, elbow joint implants, wrist joint implants, ankle joint implants, pubic joint implants, metatarsal joint implants, or other joint implants, spinal implants, spinal implants and intervertebral disc implants, radial head implants, thumb implants, and implants for osteotomy (high tibial osteotomy). Through the surface treatment according to the present disclosure, an antibacterial effect may be provided to the application site of the orthopedic implants, thereby enabling the use thereof as implants in the heavy-load area of joints and endo-osseous prostheses.

For example, the orthopedic implant may be a joint implant, for example, a hip joint implant and a knee joint implant. The implant of the hip joint may be: a dysplasia head and prosthesis (endoosseous prosthesis or interosseous of the femur) and a cortiloid cavity prosthesis. A knee joint implant may include the tibial and femoral components of the knee joint.

For example, dental implants include dental implants themselves (spiral, cylindrical, conical or layered), dental abutments, integrated abutments, dental crowns, dental implants in which abutments and crowns are integrated, dental beams, dental inserts, and dental pins.

In an embodiment, the surface treatment method of the implant may be applied to artificial bone, bone preservation material, etc., and the artificial bone or bone preservation material may be used to supplement a bone defect caused by a fracture or tumor resection or cartilage removed by lumbar spine surgery. For example, the implant may be an artificial hip joint embedded in the femur.

In an embodiment, the surface treatment method of the implant may be applied to a member of an artificial joint, a bone junction material used for fixing a fracture site, a fixing device for a spine (spine implant or lumbar implant), and the like.

The surface treatment method of the implant of the present disclosure is not limited to implants embedded in the living body (in vivo), and may also be applied to implants fixed to the body surface. The surface treatment method of the implant of the present disclosure may be applicable to pets or livestock, not limited to humans.

Detailed description of the implant and surface treatment thereof may be the same as or applicable mutatis mutandis as described in connection with the implant structure.

Another aspect provides: an antibacterial composition including a metal material having, on the surface thereof, a plurality of protrusions having an aspect ratio of 1:1 to 1:50, wherein the metal material includes titanium or a titanium alloy, and is surface-treated with a femtosecond laser; and an antibacterial method including bringing the antimicrobial composition including the metal material into contact with an object.

Detailed description of the implant and surface treatment thereof may be the same as or applicable mutatis mutandis as described in connection with the implant structure.

ADVANTAGEOUS EFFECTS OF DISCLOSURE

The implant structure according to an aspect has the surface having the first outer peripheral surface and the second outer peripheral surface, each having a specific pattern including nano-protrusions, and the characteristics of these outer peripheral surfaces may improve the bondability between the implant structure and the bone tissue, and the antimicrobial properties of the implant. Therefore, the implant structure according to an aspect can reduce side effects of the conventional cases, such as inflammation, caused by an implant having a biofilm applied to the surface, thereby providing excellent biocompatibility and stability.

BRIEF DESCRIPTION OF DRAWINGS

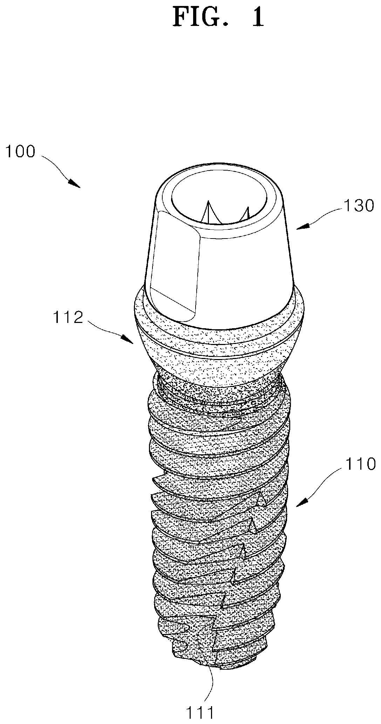

FIG. 1 is a perspective view of an implant structure including an abutment-integrated fixture according to an embodiment of the present disclosure.

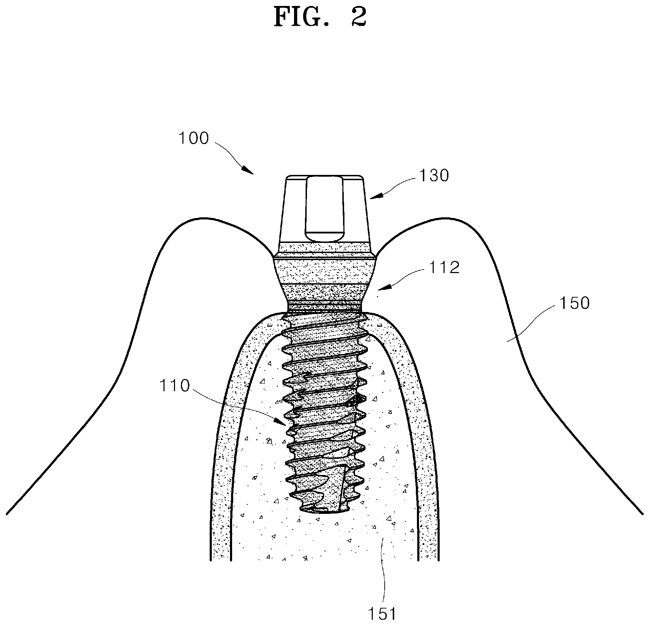

FIG. 2 illustrates an implant structure including a fixture according to an embodiment of the present disclosure, which is inserted into an alveolar bone.

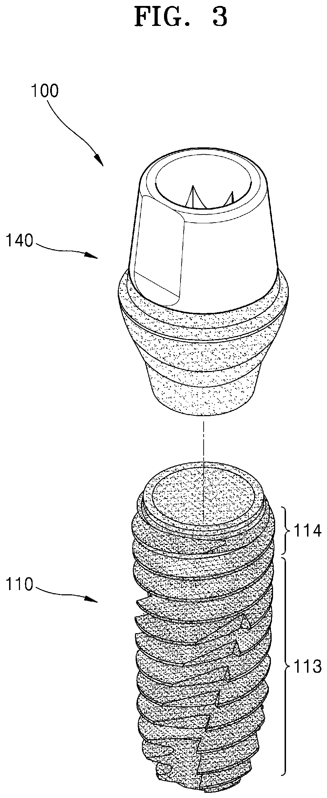

FIG. 3 is a perspective view of an implant structure including an abutmentdetachable fixture according to an embodiment of the present disclosure.

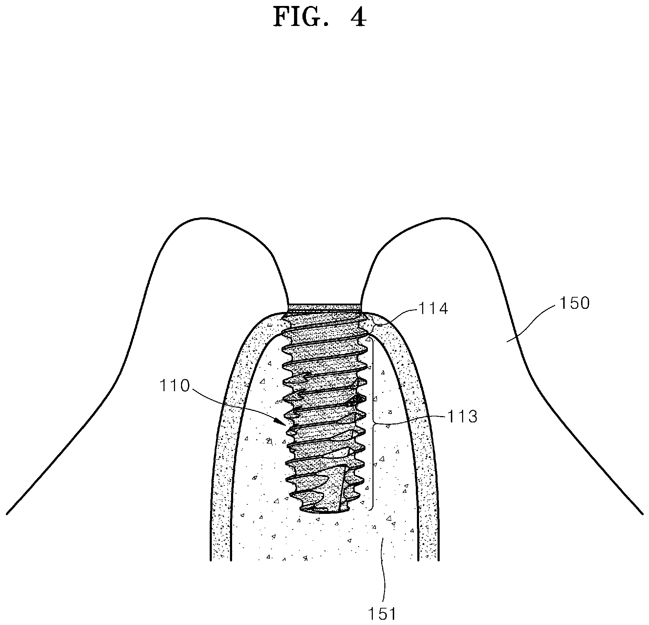

FIG. 4 illustrates a fixture according to an embodiment of the present disclosure, which is inserted into an alveolar bone.

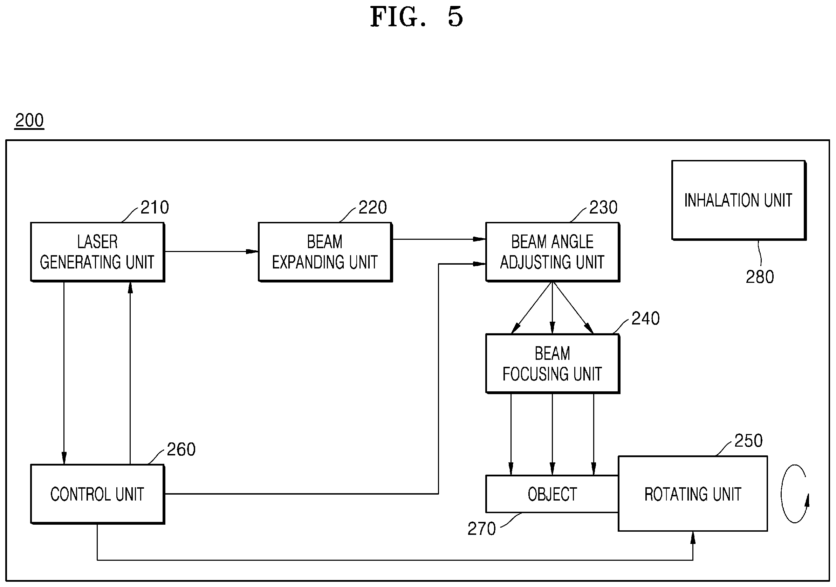

FIG. 5 illustrates a system for manufacturing an implant structure according to the present disclosure.

FIG. 6 shows images of the surface of a titanium implant structure: (A) of FIG. 6 shows an image of the titanium which is not surface-treated; (B) of FIG. 6 shows an image of the surface of the titanium metal implant which is treated with SLA (Sandblasted, Large grit, Acid-etched); (C) of FIG. 6 shows an image of the surface of titanium which is surface-treated with a femtosecond laser, and (D) of FIG. 6 shows images of the surface of the titanium that is surface-treated and the surface of the titanium that is surface-treated with a femtosecond laser.

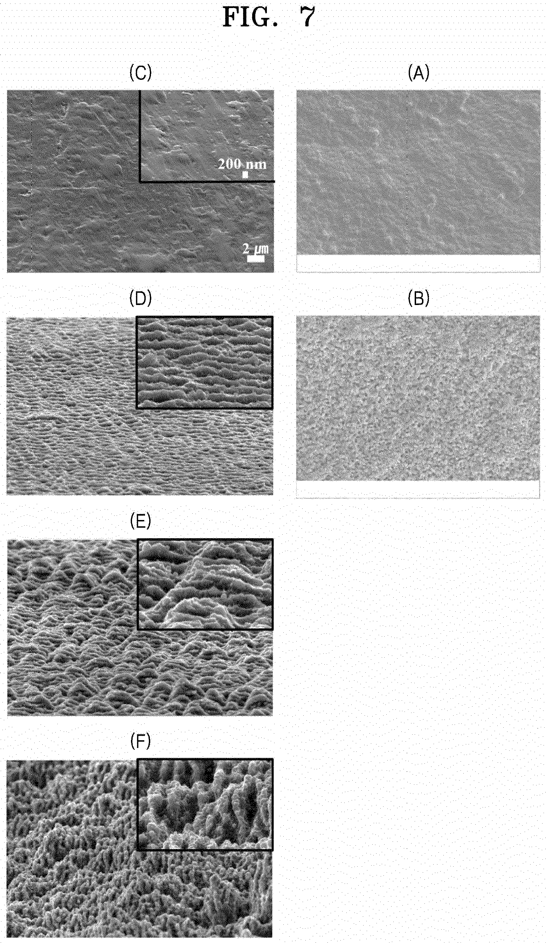

FIG. 7 shows enlarged images of the surface of a titanium metal implant and the surface of a zirconia implant structure according to the surface-treatment: (A) of FIG. 7 shows the image of the zirconia surface that is not surface-treated; (B) of FIG. 7 shows the image of the zirconia surface that is surface-treated with Laser 1; (C) of FIG. 7 shows the image of the titanium surface that is not surface-treated; (D) of FIG. 7 shows the image of the titanium surface that is surface-treated with Laser 1; (E) of FIG. 7 shows the image of the titanium surface that is surface-treated with Laser 2; and (F) of FIG. 7 shows the image of the titanium surface that is surface-treated with Laser 3.

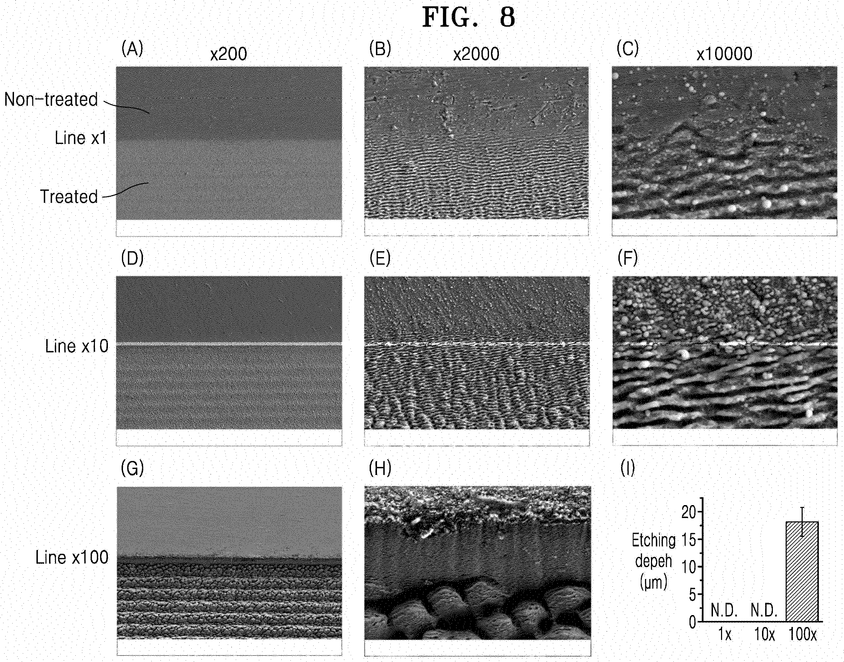

FIG. 8 shows enlarged images of the surface of the titanium metal implant structure that is treated linearly using a femtosecond laser while the number of processing varies: (A), (B), and (C) of FIG. 8 show images of the surface of the metal implant structure that is linearly processed once; (D), (E), and (F) of FIG. 8 show images of the surface of the metal implant structure that is linearly processed 10 times; (G) and (H) of FIG. 8 show images of the surface of the metal implant structure that is processed 100 times; and (I) of FIG. 8 shows the graph of the depth of the metal implant structure processed 100 times.

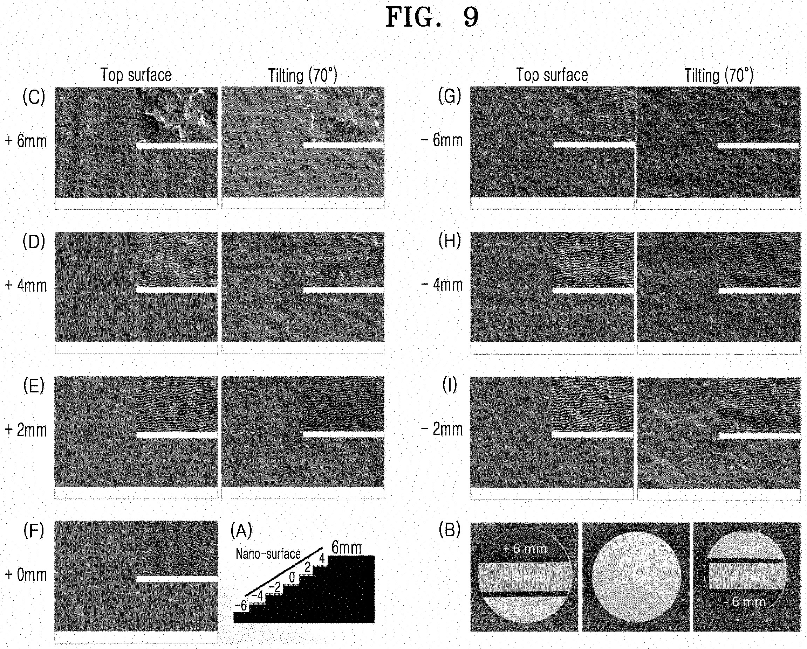

FIG. 9 shows images of the implant structure which is surface-treated with different femtosecond laser surface treatment conditions, indicating that even when the portion to be surface treated is a certain distance away from the laser focus area during the surface treatment using a femtosecond laser, the surface treatment is able to be performed.

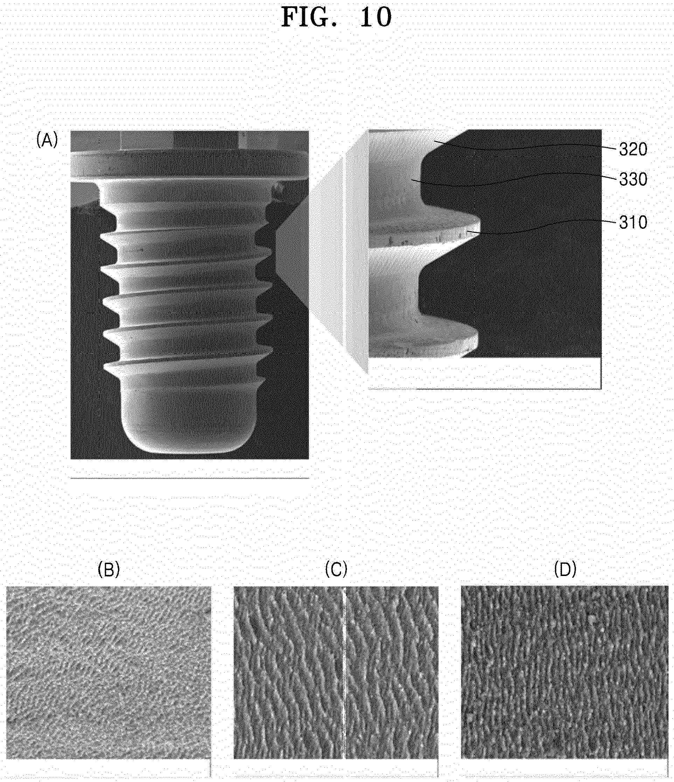

FIG. 10 shows images of the metal implant structure which is surface-treated with a femtosecond laser: (A) of FIG. 10 shows the surface of the first outer peripheral surface including a thread upper surface 310, a thread lower surface 320, and a thread groove side surface 330; (B) of FIG. 10 shows the image of the thread upper surface 310 after the surface treatment; (C) of FIG. 10 shows the image of the thread lower surface 320 after the surface treatment; and (D) of FIG. 10 shows the image of the thread groove side surface 330 after the surface treatment.

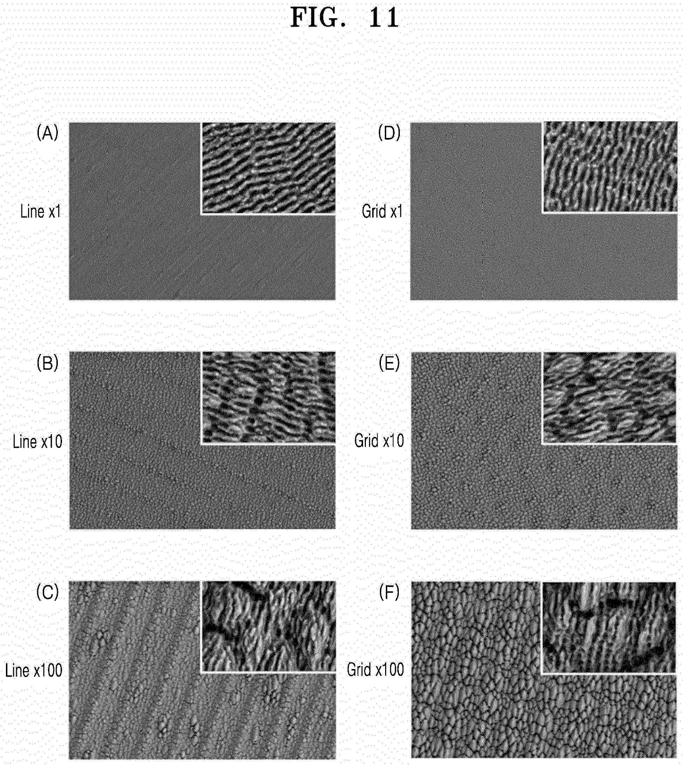

FIG. 11 shows a top view of the surface of the implant structure which is treated with a femtosecond laser while the processing type and the number of processing vary: (A) of FIG. 11 shows the case of a linear surface treatment once; (B) of FIG. 11 shows the case of a linear surface treatment 10 times; (C) of FIG. 11 shows the case of a linear surface treatment 100 times; (D) of FIG. 11 shows the case of a grid surface treatment once; (E) of FIG. 11 shows the case of a grid surface treatment 20 times; and (F) of FIG. 11 shows the case of a grid surface treatment 100 times.

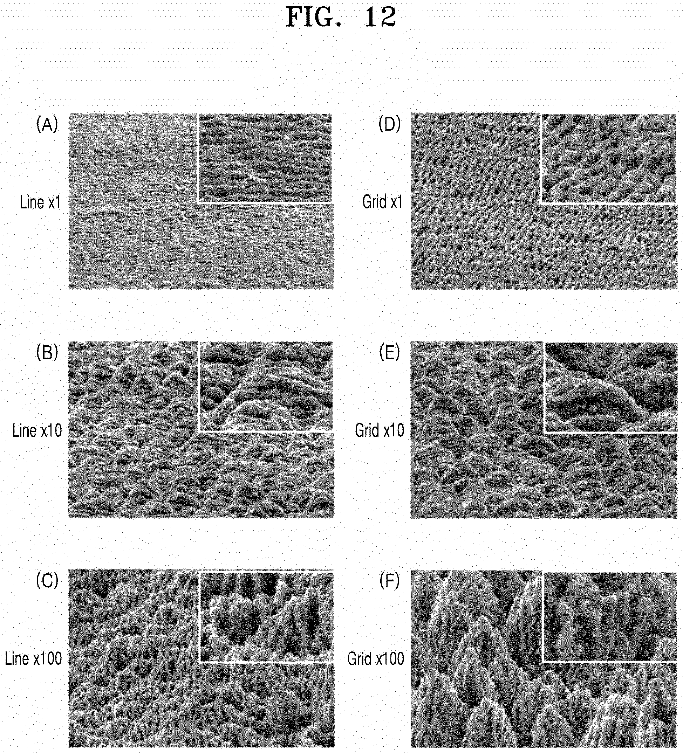

FIG. 12 shows images of the tilted surface of the implant structure which is treated with a femtosecond laser while the processing type and the number of processing vary: (A) of FIG. 12 shows the case of a linear surface treatment once; (B) of FIG. 12 shows the case of a linear surface treatment 10 times; (C) of FIG. 12 shows the case of a linear surface treatment 100 times; (D) of FIG. 12 shows the case of a grid surface treatment once; (E) of FIG. 12 shows the case of a grid surface treatment 20 times; and (F) of FIG. 12 shows the case of a grid surface treatment 100 times.

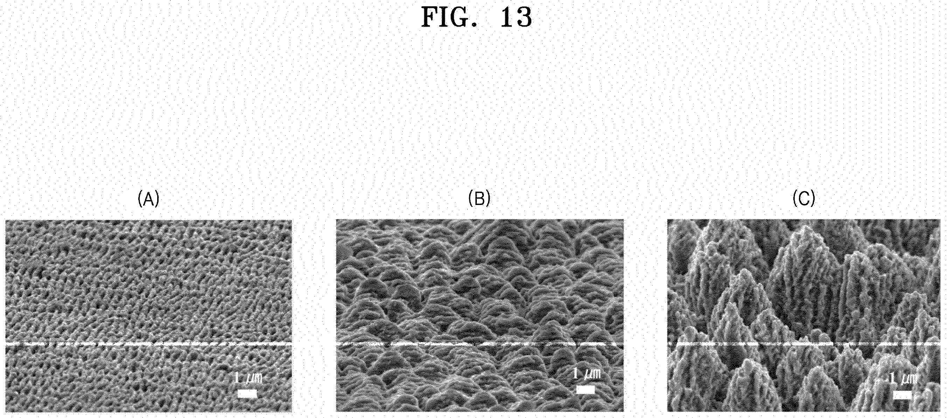

FIG. 13 shows images of nano-protrusions formed on the surface of the implant structure while the processing condition is changed by using a femtosecond laser: (A) of FIG. 13 shows the image of nano-protrusions having the aspect ratio of 100:1; (B) of FIG. 13 shows the image of nano-protrusions having the aspect ratio of 1:1; and (C) of FIG. 13 shows the image of nano-protrusions having the aspect ratio of 1:20.

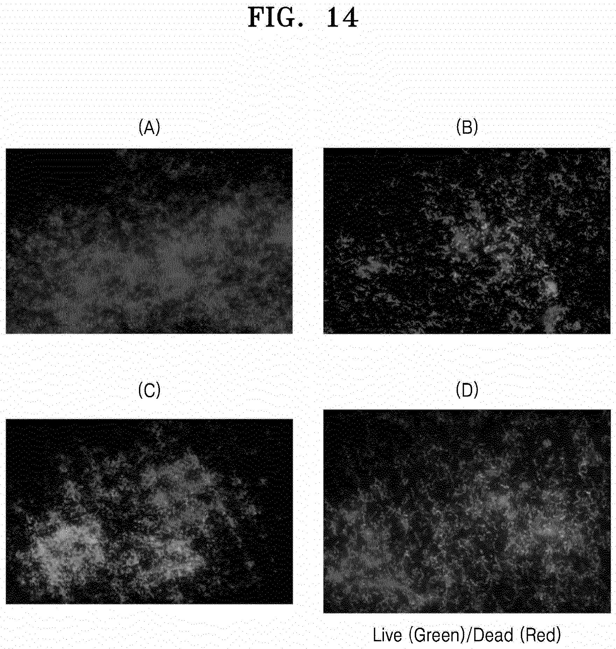

FIG. 14 shows the effect of killing bacteria according to the shape of the surface of the implant structure: (A) of FIG. 14 shows the effect of killing bacteria in Control; (B) of FIG. 14 shows the effect of killing bacteria in Example 1; (C) of FIG. 14 shows the effect of killing bacteria in Example 2; and (D) of FIG. 14 shows the effect of killing bacteria in Example 3.

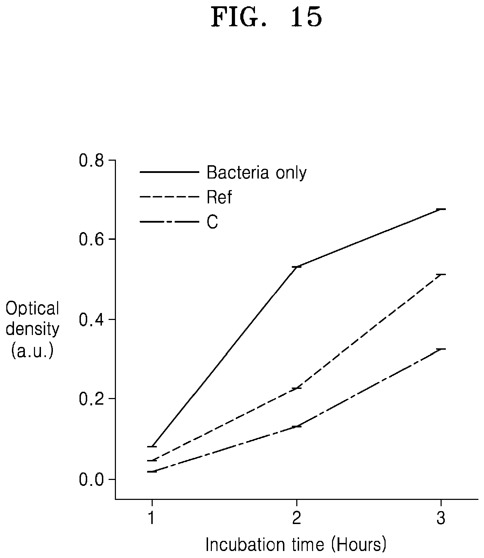

FIG. 15 shows the bacterial killing effect according to the surface shape of Example 2 according to the incubation time.

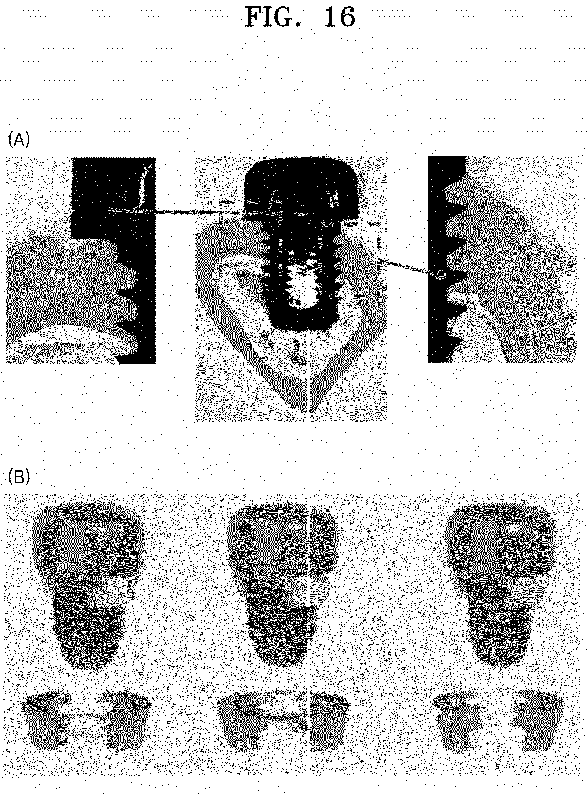

FIG. 16 shows images of a surface-treated implant structure implanted in an animal model and a computerised tomography (CT) scan result thereof: (A) of FIG. 16 shows a surface-treated implant structure implanted in an animal model; and (B) of FIG. 16 shows CT scan results of an implant structure which is surface-treated by simple machining, an implant structure which is surface-treated by a femtosecond laser, and an implant structure which is treated with a SLA (Sandblasted, Large grit, Acid-etched) surface treatment.

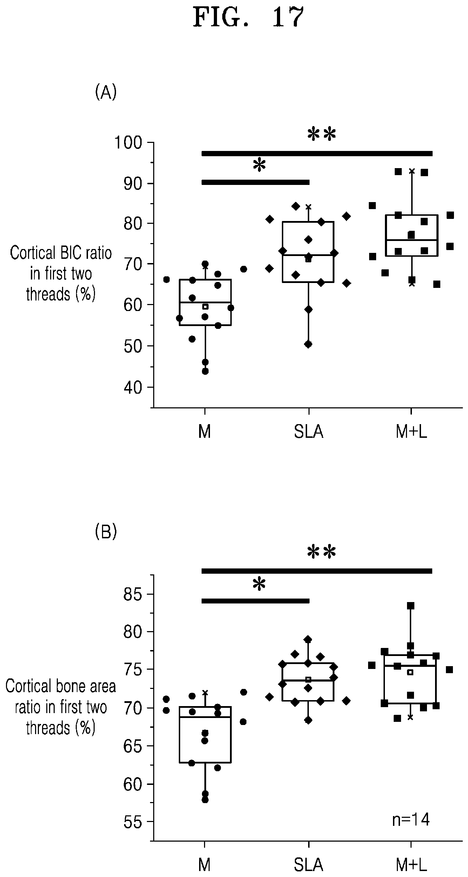

FIG. 17 shows the diagram with which the osseointegration effect of a surface-treated implant structure implanted in an animal model was confirmed: (A) of FIG. 17 shows the graph of the cortical BIC ratio; and (B) of FIG. 17 shows the graph of the cortical bone area ratio.

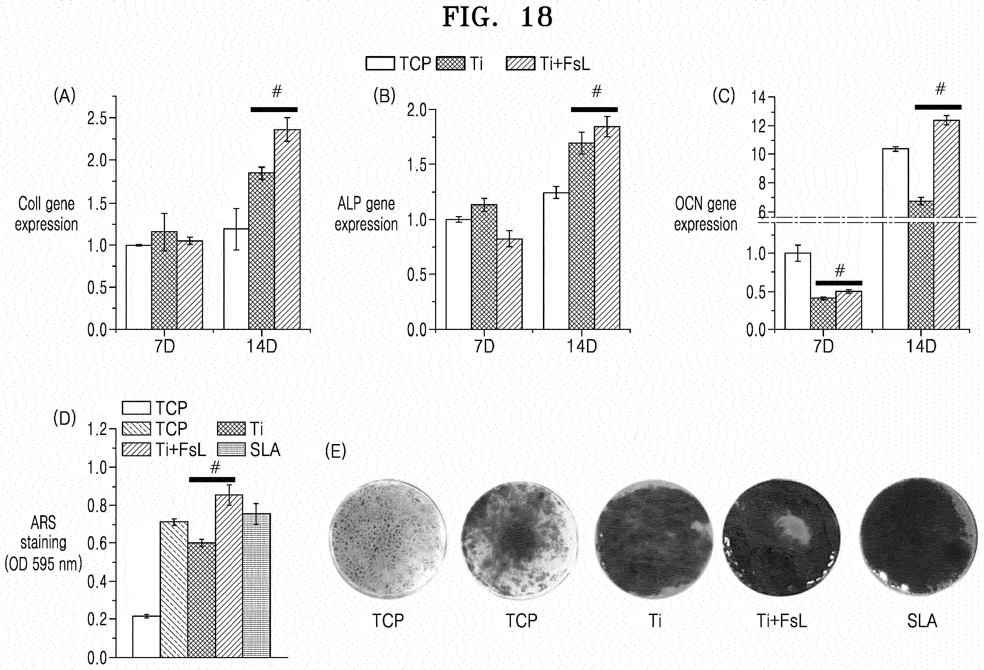

FIG. 18 shows diagrams related to the expression and cell differentiation of bone formation-related genes according to the surface treatment of an implant structure in a stem cell differentiation experiment: (A) of FIG. 18 shows the graph of the expression of Col1 gene; (B) of FIG. 18 shows the graph of the expression of the ALP gene; (C) of FIG. 18 shows the graph of the expression of the OCN gene; (D) of FIG. 18 shows a quantitative comparison of the cell differentiation level by ARS staining; and (E) of FIG. 18 shows the result obtained visually confirming the ARS staining level.

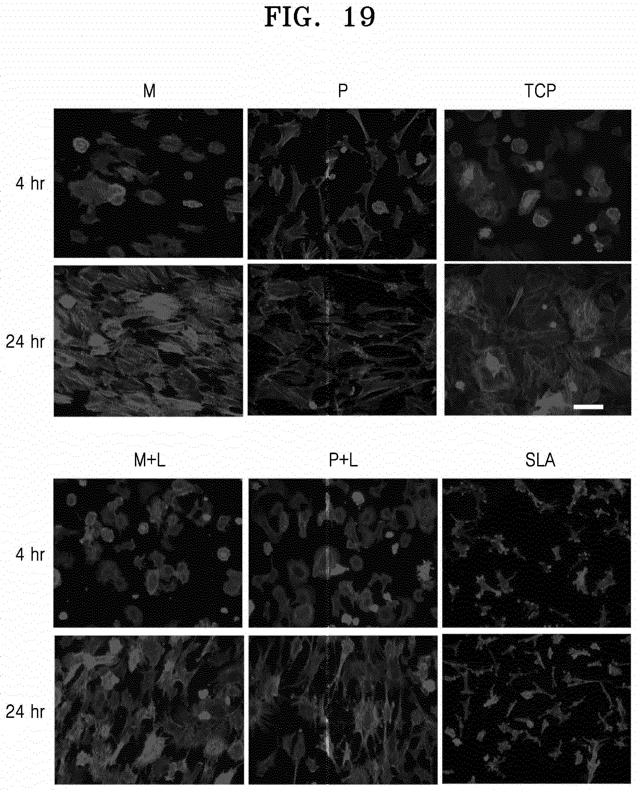

FIG. 19 shows a result of confirming the cell adsorption capacity of the surface-treated implant structure through fluorescence staining.

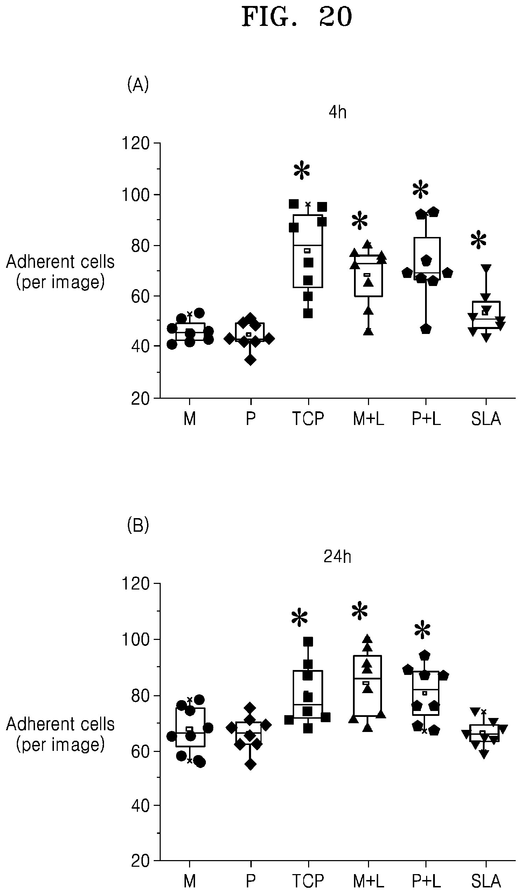

FIG. 20 shows a diagram to quantitatively compare the cell adsorption capacity of the surface-treated implant structure: (A) of FIG. 20 shows the diagram of the cell adsorption capacity at 4 hours of culture; and (B) of FIG. 20 shows the results of comparing the cell the diagram of the cell adsorption capacity at 24 hours of culture.

MODE OF DISCLOSURE

Hereinafter, the configuration and operation of the embodiments of the present disclosure will be described in detail with reference to the accompanying drawings.

The following examples are provided to more completely explain the disclosure to those of ordinary skill in the art, and the following examples may be modified in various other forms, and the scope of the disclosure is not limited to the following examples.

The terminology used herein is used to describe specific embodiments, not to limit the disclosure. As used herein, the singular form may include the plural form unless the context clearly dictates otherwise. Also, the terms "comprise, include" and/or "comprising, including" are used to specify the presence of the referenced shapes, numbers, processes, actions, members, elements, and/or groups thereof, and do not exclude the presence or addition of one or more other shapes, numbers, actions, members, elements, and/or groups.

In the present specification, although an "implant fixture" is described as an example of the implant structure, the present disclosure is not limited thereto, and includes all implants that are able to be subjected to the surface treatment. In an embodiment, in this specification, "beam angle adjusting unit" is used as a generic term for the two-dimensional scan mirror unit and the single-axis scan mirror unit, and "rotating unit" is used as a general term for the stepping rotating unit and the continuous rotating unit.

1. Implant structure

The present disclosure provides an implant structure including a fixture that acts as an artificial tooth root, wherein the fixture includes a bone contact portion 111 having a first outer peripheral surface in which a thread is formed and a gingival contact portion 112 having a second outer peripheral surface, disposed above the second outer peripheral surface, wherein the first outer peripheral surface or the second outer peripheral surface includes nano-protrusions having a width of 10 nm to 1000 nm and an aspect ratio of 1000:1 to 1:50.

The present disclosure provides an implant structure including a fixture that acts as an artificial tooth root, wherein the fixture includes a bone contact portion 111 including an outer peripheral surface having a thread formed thereon, and the outer peripheral surface includes a bone formation facilitation region 113 including a plurality of nano-protrusions having a width of 10 nm to 1000 nm and an aspect ratio of 1000:1 to 1:1, and an antimicrobial activity facilitation region 114 located on the bone formation facilitation region and including a plurality of nano-protrusions having a width of 10 nm to 1000 nm and an aspect ratio of 1:1 to 1:50.

FIGS. 1 to 4 illustrate an example structure and shape of an implant structure according to an embodiment, and an image of the implant structure being inserted into an alveolar bone.

The fixture 110 of the implant structure 100 according to the present disclosure is implanted in a missing alveolar bone to form a tooth root, and may include a bone contact portion 111, a gingival contact portion 112, and an upper portion 130.

The fixture 110 of the implant structure 100 according to the present disclosure may be implanted in the missing alveolar bone to form a tooth root, and may include the bone contact portion 111 including a bone formation facilitation region 113 and an antimicrobial activity facilitation region 114.

The fixture 110 of the implant structure 100 may include a material which is harmless to the human body, for example, titanium, and the surface of the fixture 110 may be subjected to a certain surface treatment. The fixture 110 of the implant structure is generally called an abutment (not shown) and may be classified into various types depending on whether the abutment to which the artificial teeth are coupled is integrally formed. In an embodiment, the abutment 140 may be provided as an integral implant fixture in which the abutment 140 is integrally formed with the fixture. In an embodiment, the abutment 140 may be manufactured as being separated from the fixture, and may be coupled when the implant structure is inserted.

The fixture 110 of the implant structure 100 described in an embodiment may be the fixture for the abutment-integrated implant of FIG. 1 or the fixture for the abutmentseparated implant of FIG. 3.

For example, the bone contact portion 111 is a portion of the fixture 110 of the implant structure 100 which is inserted into an alveolar bone 151, and may include a first outer peripheral surface in which a thread wound in a spiral shape is formed on the outer peripheral surface of a cylindrical shape thereof as a whole. The cylindrical shape may be a cylindrical pillar shape or a truncated cone shape, and the truncated cone shape may have a wider upper portion and a narrower lower portion, and vice versa. The thread may be a single-lead screw or a double-lead screw, and in other cases, may be a multilead screw. At this time, the pitch of each screw may be the same. The diameter of the thread may be gradually increased and, after a certain section, the diameter may be maintained constant. In an embodiment, a cutting blade (not shown) for self-tapping may be formed at the lower end thereof, and the cutting blade may allow the fixture 110 to be easily inserted while cutting the peripheral bone of the alveolar bone during the initial insertion process.

The thread may include a thread upper surface 310, a thread lower surface 320, and a thread groove side surface 330.

The first outer peripheral surface of the bone contact portion 111 has nano-protrusions having a specific aspect ratio formed on the surface. For example, a specific pattern by nano-protrusions may be prepared by a laser surface treatment. The surface treatment may be performed by simultaneously performing a blasting process, in which sand fine particles are sprayed to increase the surface roughness, and an etching process, in which an etching solution is used to corrode the surface to increase the surface roughness.

The bone contact portion 111 may be exposed to bacteria, etc. However, the bone contact portion 111 may be mainly inserted into the alveolar bone 151 and requires bone fusion, and may be surface-treated to promote bone fusion.

The bone contact portion 111 may include the bone formation facilitation region 113 corresponding to the first outer peripheral surface and the antibacterial activity facilitation region 114 corresponding to the second outer peripheral surface. In this case, the risk of exposure to bacteria is reduced and at the same time, and risks caused by the exposure to bacteria, etc., which may occur at the upper part of the bone contact portion 111, may be reduced.

Due to the laser surface treatment, the surface pattern of each of the thread upper surface 310, the thread lower surface 320, and the thread groove side surface 330 of the bone contact portion 111 may be treated differently.

The bone contact portion 111 may be processed to form an osseointegration facilitation structure on the thread lower surface 320 and a blunt structure on the thread upper surface 310. In an embodiment, the osseointegration facilitation structure and the blunt structure may all be formed in the thread groove side surface 330.

The gingival contact portion 112 may include a second outer peripheral surface which is disposed above the bone contact portion 111 and has an outer peripheral surface on which the thread is not formed. The gingival contact portion 112 may contact a gingiva 150.

The gingival contact portion 112 may have a cylindrical, conical, or layered shape, and for example, may be cylindrical (for example, a cylindrical pillar shape, a truncated shape), and the truncated cone shape may have a wider upper portion and a narrower lower portion, and vice versa.

The second outer peripheral surface of the gingival contact portion 112 has nano-protrusions having a specific aspect ratio formed on the surface. For example, a specific pattern by nano-protrusions may be prepared by a laser surface treatment. The surface treatment may be performed by simultaneously performing a blasting process, in which sand fine particles are sprayed to increase the surface roughness, and an etching process, in which an etching solution is used to corrode the surface to increase the surface roughness.

However, although the gingival contact portion 112 needs bone fusion when being inserted into the alveolar bone 151, the gingival contact portion 112 is highly likely to have a contact with the gingiva 150 and to be exposed to, for example, bacteria. Accordingly, the gingival contact portion 112 needs to be surface-treated to facilitate the depth of bacteria existing on the surface.

The upper portion 130 may be disposed above the gingival contact portion 112, and may be coupled to an abutment or a crown depending on the type of implant. For example, in the case of an integrated implant fixture, a crown may be coupled to the upper portion thereof, and in the case of an implant structure in which the fixture and the abutment are separated from each other, the abutment may be coupled to the upper portion thereof.

The upper portion 130 may have a substantially truncated cone shape. The upper portion 130 may have a smooth surface formed by simply cutting, without the surface treatment.

2. Implant structure manufacturing system

2.1. Case in which an object is a rotating body

FIG. 5 illustrates a system for manufacturing an implant structure according to the present disclosure.

Referring to FIG. 5, a laser surface treatment apparatus 200 according to an embodiment of the present disclosure may include a laser generating unit 210, a beam expanding unit 220, a beam angle adjusting unit 230, and a beam focusing unit 240, a rotating unit 250, and a control unit 260. The components shown in FIG. 3 are not essential in implementing the implant structure manufacturing system, so the implant structure manufacturing system described herein may have more or fewer components than those listed above.

The laser generating unit 210 may generate a laser beam for the surface-treatment of the object 270. To this end, the laser generating unit 210 may use a pulsed laser source. The laser beam generated in pulse units by the laser generating unit 210 may be irradiated onto the object 270 sequentially through the beam expanding unit 220, the beam angle adjusting unit 230, and the beam focusing unit 240.

The laser generating unit 210 may generate a laser beam of any one of nanoseconds, picoseconds, and femtoseconds. In an embodiment, the laser generating unit 210 may generate a femtosecond laser beam. Here, the femtosecond laser beam is an ultrashort laser beam having a pulse duration time of 1 femtosecond to 1000 femtoseconds. Accordingly, the laser generating unit 210 according to an embodiment of the present invention may generate a pulsed laser beam having a pulse duration time of the femtoseconds range. Here, the pulse repetition rate may be in the range of several to several hundreds kHz or in the MHz range. As the wavelength of the laser beam, all laser wavelengths located within the range from the infrared region to the ultraviolet region may be used. For example, the wavelength of the laser beam may include an infrared wavelength, a visible light wavelength, and an ultraviolet wavelength.

The beam expanding unit 220 may adjust the size of the laser beam generated by the laser generating unit 210 in units of pulses. More specifically, the beam expanding unit 220 may expand the size of the laser beam. In an embodiment, the beam expanding unit 220 may generate a laser beam as a collimated beam with less dispersion or focusing. Accordingly, the laser beam generated by the laser generating unit 210 may be converted into a collimated beam while being enlarged in size, through the beam expanding unit 220.

The beam expanding unit 220 may change a diameter of the laser beam generated by the laser generating unit 210 and output the changed laser beam toward the beam angle adjusting unit 230. In this case, the beam expanding unit 220 may manually or automatically adjust the size of the laser beam.

The beam angle adjusting unit 230 may adjust the focal position of the laser beam output by the beam expanding unit 220. The beam angle adjusting unit may be a two-dimensional scan mirror unit or a single-axis scan mirror unit.

The two-dimensional scan mirror unit (or XY scan mirror unit) is a beam angle adjusting unit configured to have two axes, that is, the two-dimensional scan mirror unit, a laser beam irradiated to the object 270 may adjust the focal position of the laser beam irradiated to the object 270, that is, the focal position on the X-axis and Y-axis, according to a control signal from the control unit 260. The single-axis scan mirror unit is a beam angle adjusting unit configured to have one axis, may adjust the focal position of a laser beam output from the beam expanding unit 220. That is, the single-axis scan mirror unit may adjust the focal position of the laser beam irradiated to the object 270, that is, the focal position on the X-axis or the Y-axis, according to a control signal from the control unit 260.

The beam angle adjusting unit 230 may include an X-axis scan mirror and a Y-axis scan mirror to perform a one-dimensional or two-dimensional scanning operation. Here, each of the X-axis scan mirror and the Y-axis scan mirror is configured to include a pair of scan mirrors having a galvanometer method, and each of the pair of scan mirrors may deflect the laser beam in one of directions of axes which transverse on the X-Y plane.

The beam angle adjusting unit 230 may reflect the laser beam in a direction for laser surface treatment through the X-axis scan mirror and the Y-axis scan mirror to irradiate the laser beam to a target position on the object 270. In an embodiment, the beam angle adjusting unit 230 may finely control the laser beam in the direction of X-axis or Y-axis along the upper surface of the object 270, through the X-axis scan mirror and the Y-axis scan mirror.

For example, in an embodiment, the beam angle adjusting unit 230 may irradiate the laser beam on the first outer peripheral surface or the second outer peripheral surface of the implant structure 100, which corresponds to an object, in a horizontal direction (that is, the longitudinal direction of implant structure), along a predetermined scanning path, according to a control signal from the control unit 260.

Here, nano-protrusions may be formed on the first outer peripheral surface or the second outer peripheral surface of the implant structure 100. In an embodiment, the predetermined scanning path may be determined based on two-dimensional focal position data provided from the control unit 260 to the beam angle adjusting unit 230.

Meanwhile, in an embodiment, the surface of the first outer peripheral surface or the second outer peripheral surface of the implant structure 100 which is treated with a laser beam may be at the higher or lower levels than a portion which is not surface-treated. In an embodiment, the predetermined scanning path may be determined based on two-dimensional focal position data provided from the control unit 260 to the beam angle adjusting unit 230.

The beam focusing unit 240 may be disposed under the beam angle adjusting unit 230 to focus, to the object 270, the laser beam which has passed through the beam angle adjusting unit 230.

Also, the beam focusing unit 240 may change a laser beam incident from the beam angle adjusting unit 230 at a certain angle into a direction perpendicular to the longitudinal direction of the object 270.

The beam focusing unit 240 may include a telecentric F-theta lens or an F-theta lens. Accordingly, the beam focusing unit 240 may perform a micro- or nano-scale laser surface treatment on the surface of the object 270 (for example, the first outer peripheral surface or the second outer peripheral surface).

The rotating unit 250 may fix the object 270 such that the object 270 is positioned in a direction in which the laser beam travels, that is, under the beam focusing unit 240. In an embodiment, the rotating unit 250 may rotate, according to a control signal from the control unit 260, the object 270 by a predetermined angle (for example, 120 degrees) for each surface treatment process.

The control unit 260 may control the overall operation of the laser surface treatment apparatus 200. In an embodiment, the control unit 260 may control at least some of the components 210 to 250 in order to drive an application program stored in a memory. Furthermore, the control unit 260 may, to drive the application program, combine at least two or more of the components included in the laser surface treatment apparatus 200 and operate the same.

More specifically, the control unit 260 may control the laser generating unit 210 to adjust the wavelength, pulse duration time, and pulse repetition rate of the laser beam generated in units of pulses by the laser generating unit 210.

In an embodiment, the control unit 260 may generate a control signal including two-dimensional focal position data (that is, focal position data of X and Y axes) of a laser beam for treating the surface of the object 270, and may transmit the control signal to the beam angle adjusting unit 230. The beam angle adjusting unit 230 may irradiate the laser beam based on the two-dimensional focus position data included in the control signal transmitted by the control unit 260.

In an embodiment, the control unit 260 monitors the surface treatment process using a laser on one area of the object 270 in real time, and when the surface treatment for the one area is completed, the control unit 260 controls the rotating unit 250 to rotate the object 270 by a predetermined angle step by step, and then control the laser surface treatment for other areas of the object 270.

For example, the control unit 260 may control the laser generating unit 210 and the beam angle adjusting unit 230 to perform the laser surface treatment on the first outer peripheral surface of the fixture 110 of the implant structure 100. When the surface treatment of the first outer peripheral surface is completed, the control unit 260 controls the rotating unit 250 to rotate the fixture 110 of the implant structure 100 in a clockwise or counterclockwise direction by a predetermined angle (for example, 120 degrees), and then, controls the laser generating unit 210 and the beam angle adjusting unit 230 to perform the laser surface treatment on the second outer peripheral surface of the fixture 110 of the implant structure 100. When the surface treatment of the second outer peripheral surface is completed, the control unit 260 controls the rotating unit 250 to rotate the fixture 110 of the implant structure 100 in the same direction by a predetermined angle (for example, 120 degrees), and then, controls the laser generating unit 210 and the beam angle adjusting unit 230 to perform the laser surface treatment on the surface of an abutment of the implant structure 100. Through this step-by-step rotation process, the surface of the implant structure 100 may be surface-treated at a very high speed.

Meanwhile, as another example, depending on the shape of the object, the object may undergo more or less the surface treatment processes than the surface treatment process described above. In an embodiment, the rotating may be configured to be made by different angles step by step rather than the same angle step by step.

The control unit 260 may control such that the laser generating unit 210, the beam angle adjusting unit 230, and the rotating unit 250 are synchronized with each other. That is, the control unit 260 may control the beam angle adjusting unit 230 based on the generation period of the laser beam irradiated from the laser generating unit 210. In an embodiment, the control unit 260 may control the rotating unit 250 by synchronizing with the time for surface-treating the object 270 through the laser generating unit 210 and the beam angle adjusting unit 230.

In an embodiment, the laser surface treatment apparatus 200 may include an inhalation unit 280 for sucking fine particles generated while performing the surface treatment of the implant structure. The inhalation unit 280 may prevent the fine particles generated during processing from adhering to and accumulating on the base material.

As described above, the laser surface treatment apparatus according to an embodiment of the present disclosure may perform the laser surface treatment on an object at a very high speed by using a beam angle adjusting unit and a rotating unit. By treating the surface at such a high speed, the overall process time for laser surface treatment can be shortened.

2.2. Case in which an object is not a rotating body

When the implant structure is not a rotating body, the laser surface treatment apparatus (not shown) may include a laser generating unit, a beam expanding unit, a beam angle adjusting unit, a beam focusing unit, an object moving unit, and a control unit.

In this case, the implant structure may be fixed on an object moving unit and processed. The object moving unit may be synchronized with the surface treatment time of the laser beam by the control unit, and may move the object horizontally or vertically.

Due to the horizontal or vertical movement of the object, the surface of the object may be surface-treated by a laser beam.

Except for the object moving unit, the laser generating unit, the beam expanding unit, the beam angle adjusting unit, the beam focusing unit, and the control unit of the laser surface treatment apparatus are applied mutatis mutandis as described in connection with the case where the object is a rotating body.

Experimental Example 1. Confirmation of the surface of the implant structure according to the surface treatment method

FIG. 6 illustrates a titanium implant structure: an implant structure which is not surface-treated ((A) of FIG. 6); an implant structure which is surface- treated with SLA(Sandblasted, Large grit, Acid-etched) ((B) of FIG. 6); and an implant structure which is surface-treated with a femtosecond laser beam ((C) of FIG. 6).

As shown in (A) of FIG. 6, it was confirmed that no specific structure or pattern was formed on the surface of the titanium implant structure that was not subjected to the surface treatment.

As shown in (B) of FIG. 6, when the implant structure was surface-treated through SLA, it was confirmed that an irregular pattern was formed on the surface of the implant structure.

In an embodiment, the implant structure was surface-treated with a femtosecond laser beam and the surface thereof was confirmed. At this time, the energy of the femtosecond laser was 1.2 J/cm$^2$, the pulse width was 230 fs, and the pulse repetition rate was 100 kHz. As a result, as shown in (C) of FIG. 6, it was confirmed that a certain pattern was formed on the surface of the implant structure.

Experimental Example 2. Surface treatment using a femtosecond laser

The surface of the implant structure was treated by controlling the size of the area to be processed, the processing speed and the processing direction, etc. by setting the processing conditions of the femtosecond laser differently. The processing speed was adjusted to be from 0.1 mm/s to 1000 mm/s, and the processing shape was treated as a point type, a linear type, or a grid type.

FIG. 7 shows enlarged images of the surface of a titanium metal implant and the surface of a zirconia implant structure according to the surface-treatment: As shown in FIG. 7(B), in the case of the zirconia implant structure, a uniform pattern was not formed during surface treatment. On the other hand, (D) to (F) of FIG. 7, in the case of the titanium metal implant, it was confirmed that a certain pattern was formed during the surface treatment. At this time, the femtosecond laser processing conditions are shown in Table 1 below.

[Table 1]

|  | Laser 1 | Laser 2 | Laser 3 |
|---|---|---|---|
| Laser power (W) | 1.5 | 1.5 | 1.5 |
| Processing speed (mm/s) | 500 | 500 | 500 |
| Processing shape | Line | Line | Line |
| Number of processing (times) | 1 | 10 | 100 |

FIG. 8 shows enlarged images of the surface of the titanium metal implant structure that is treated linearly using a femtosecond laser while the number of processing varies. As a result, it was confirmed that the pattern was formed in a certain shape on the surface of the implant structure as the number of processing is increased. When the surface treatment is performed linearly 100 times,, it was confirmed that when the surface treatment was linearly performed 100 times, the depth was about 170 μm. Therefore, it was confirmed that the linear heat treatment is performed once using a femtosecond laser, the etching was performed to the depth of about 170 nm.

FIG. 9 shows images of the surface of the implant structure of which height and depth varies depending on the condition of the surface treatment of the femtosecond laser. As a result, it was confirmed that the etched surface was a height of 0 mm to 10 mm higher or a depth of 0 mm to 10 mm lower than the surface which is not surface-treated according to the set processing conditions. In an embodiment, in the case of the surface treatment using a femtosecond laser, even when being away from the laser focal region by a certain distance, the surface treatment can be performed. For example, the surface treatment can be performed even when the portion to be surface-treated is 8 mm to 12 mm away from the laser focal region (4 mm to 6 mm below or 4 mm to 6 mm above the portion which is not surface-treated). Since the surface treatment can be performed as described above, it was confirmed that the thread of the implant fixture having a difference in the height of less than 10 mm can be surface- treated once or several times.

FIG. 10 shows images of the metal implant structure which is surface-treated with a femtosecond laser: (A) of FIG. 10 shows the thread upper surface 310, the thread lower surface 320, and the thread groove side surface 330 of the surface of the first outer peripheral surface in the case where the surface treatment was performed simultaneously while the linear treatment condition using a femtosecond laser was set once; (B) of FIG. 10 shows an enlarged image of the surface of the thread upper surface 310; (C) of FIG. 10 shows an enlarged image of the surface of the thread lower surface 320; and (D) of FIG. 10 shows an enlarged image of the surface of the thread groove side surface 330. As shown in FIG. 8, it was confirmed that femtosecond laser treatment may embody a surface that exhibits an osseointegration facilitation effect even on the surface of a metal implant structure having the structure including a thread and a thread groove.

FIGS. 11 and 12 show images of the surface of the implant structure which is treated using a femtosecond laser while the processing shape and the number of processing are adjusted. For example, the surface of the implant structure was treated in a linear type and a grid type by a femtosecond laser. (A) of FIG. 11 and (A) of FIG. 12 show the results of the linear surface treatment which is performed once, (B) of FIG. 11 and (B) of FIG. 12 show the results of the linear surface treatment which is performed 10 times, (C) of FIG. 11 and (C) of FIG. 12 show the results of the linear surface treatment which is performed 100 times, (D) of FIG. 11 and (D) of FIG. 12 show the results of the grid surface treatment which is performed once, (E) of FIG. 11 and (E) of FIG. 12 show the results of the grid surface treatment which is performed 20 times, and (F) of FIG. 11 and (F) of FIG. 12 show the results of the grid surface treatment which is performed 100 times. As shown in FIGS. 11 and 12, it was confirmed that the surface could be processed into different shapes depending on the processing shape applied on the surface and the number of processing. Specifically, it was confirmed that, during the linear treatment, the lines formed by nano-protrusions on the surface of the implant structure (for example, arrangement in a row or mountain range) or valleys forming the width of nano-protrusions were treated clearly according to the number of processing, and during grid-type treatment, the aspect ratio of nano-protrusions of the surface of the implant structure was increased.

FIG. 13 shows images of nano-protrusions formed on the surface of the implant structure while the processing condition is changed by using a femtosecond laser: At this time, the processing conditions using the femtosecond laser are shown in Table 2 below.

[Table 2]

|  | Example 1 | Example 2 | Example 3 |
| --- | --- | --- | --- |
| Laser power (W) | 1.5 | 1.5 | 1.5 |
| Processing speed (mm/s) | 500 | 500 | 500 |
| Processing shape | Line | Grid | Grid |
| Number of processing (times) | 1 | 10 | 100 |

As a result, it was confirmed that the laser surface-treated implants could have nano-protrusions with an aspect ratio of 1000:1 to 1:50 depending on the surface treatment conditions. For example, it was confirmed that, depending on the processing shape, the nano-protrusions has the aspect ratio of 1000:1 to 10:1 in the case of a linear type, and the aspect ratio of 1:1 to 1:50 in the case of a lattice type.

When the surface treatment conditions include the linear shape and 1 cycle (Example 1), it was confirmed that nano-protrusions with curved ends were formed, when the surface treatment conditions include the grid type and 10 cycles (Example 2), it was confirmed that the nano-protrusions with blunt ends were formed, and when the surface treatment conditions include the grid type and 100 cycles (Example 3), it was confirmed that the nano-protrusions with sharp ends were formed.

Experimental Example 3. Antibacterial effect of implant structure in animal model

FIG. 14 shows the effect of killing bacteria according to the shape of the surface of the implant structure, and FIG. 15 shows the bacterial killing effect according to the surface shape of Example 2 according to the incubation time.

As a result, it was confirmed that the implant structure having the surface containing nano-protrusions with a specific aspect ratio exhibited an antibacterial effect. For example, in the implant structure of Example 1, the number of surviving bacteria was reduced along with some bacterial killing (see (B) of FIG. 14), and in the implant structure of Example 2, the bacterial killing effect was greatly increased and excellent antibacterial properties were obtained (see (C) of FIG. 14). On the other hand, the effect of killing bacteria was hardly confirmed in the implant structure of Example 3 (see (D) of FIG. 14). In an embodiment, it was confirmed that significant antibacterial activity was experimentally confirmed from the shape of the surface of Example 2 (see FIG. 15).

This result shows that while the existing technology prevents the deposition of bacteria such as bacteria on the surface of the implant structure by coating a separate coating layer with, for example, TCP on the surface of the implant structure, in the case of the implant structure according to the present disclosure, the surface thereof includes nano-protrusions having a specific aspect ratio, and thus, bacteria is directly killed and an antibacterial effect is obtained. Through the above results, it was confirmed that the implant structure which is surface-treated by the femtosecond laser can be processed to exhibit the bacteria killing effect on the implant structure surface when inserted into the human body.

Experimental Example 4. Effects of osseointegration of implant structures in animal models

(A) of FIG. 16 shows an image of a surface-treated implant structure which is implanted in an animal model, and (B) of FIG. 16 shows CT scan results of an implant structure which is surface-treated by simple machining, an implant structure which is surface-treated by a femtosecond laser, and an implant structure which is treated with a SLA (Sandblasted, Large grit, Acid-etched)surface treatment.

FIG. 17 shows the diagram with which the osseointegration effect of a surface-treated implant structure implanted in an animal model was confirmed: (A) of FIG. 17 shows the graph of the cortical BIC ratio; and (B) of FIG. 17 shows the graph of the cortical bone area ratio. As a result, it was confirmed that the cortical BIC ratio and the cortical bone area ratio were the highest in the implant structure (M+L) which is surface-treated with a femtosecond laser. This means that the implant structure which is surface-treated with a femtosecond laser exhibits an equal or higher level of osseointegration effect than the implant structure which is surface-treated with SLA.

FIG. 18 shows diagrams related to the expression and cell differentiation of bone formation-related genes according to the surface treatment of an implant structure in a stem cell differentiation experiment: (A) of FIG. 18 shows the graph of the expression of Col1 gene; (B) of FIG. 18 shows the graph of the expression of the ALP gene; (C) of FIG. 18 shows the graph of the expression of the OCN gene; (D) of FIG. 18 shows a quantitative comparison of the cell differentiation level by ARS staining; and (E) of FIG. 18 shows the result obtained visually confirming the ARS staining level. As a result, on the 14th day of stem cell differentiation in the implant structure which is surface-treated with a femtosecond laser, the expression of Col1, ALP, and OCN genes, which are genes related to bone formation, was increased by about 20%-50%. Accordingly, it was confirmed that the expression of the genes was higher than that of other surface-treated structures (see (A) to (C) of FIG. 18). In an embodiment, as a result of confirming the degree of cell differentiation by the implant structure through ARS staining, it was confirmed that the implant structure (Ti+FsL) which is surface-treated with a femtosecond laser was higher than that of other surface-treated structures (see (D) and (E) of FIG. 18).

FIGS. 19 and 20 show images with which the cell adsorption capacity of the surface-treated implant structure was able to be confirmed. Referring to FIGS. 19 and 20, M denotes a simple machining, P denotes precision polishing after the simple machining, and L denotes a surface treatment using a femtosecond laser. As a result, it was confirmed that the initial adhesion ability of osteogenic stem cells was significantly improved on the femtosecond laser-treated surface (M+L, P+L).

Through the above results, it was confirmed that the implant structure which is surface-treated using the femtosecond laser can rapidly promote bone recovery, that is, osseointegration, occurring in the vicinity of the structure when inserted into the human body.

## Claims

1. An implant structure (100) comprising a fixture (110) that acts as an artificial tooth root, wherein the fixture includes

   a cylindrical bone contact portion (111) having a first outer peripheral surface on which a thread is formed and a gingival contact portion (112) having a second outer peripheral surface and disposed on the bone contact portion, and
   the first outer peripheral surface includes a plurality of nano-protrusions having a width of 10 nm to 1000 nm and an aspect ratio of 1000:1 to 1:1, thereby promoting osseointegration, and
   the second outer peripheral surface includes a plurality of nano-protrusions having a width of 10 nm to 1000 nm and an aspect ratio of 1:1 to 1:50, thereby promoting antibacterial activity.

2. The implant structure of claim 1, wherein the nano-protrusions have blunt ends.

3. The implant structure of claim 1, wherein an arithmetic mean roughness (Ra) value of the second outer peripheral surface is in a range of 1.0 $\mu$m to 5.0 $\mu$m.

4. The implant structure of claim 1, wherein an arithmetic mean roughness (Ra) value of the first outer peripheral surface is in a range of 0.5 $\mu$m to 3.0 $\mu$m.

**5.** The implant structure of claim 1, wherein the implant structure includes at least one material selected from titanium, or titanium alloy.

**6.** The implant structure of claim 1, wherein the implant structure is surface-treated with a femtosecond laser.

**7.** The implant structure of claim 6, wherein the surface treatment is irradiating of a femtosecond laser beam having a laser intensity of 1 W to 5 W on the surface of the first outer peripheral surface or the second outer peripheral surface in a linear-type and grid-type one or more times.

**8.** The implant structure of claim 7, wherein the first outer peripheral surface or the second outer peripheral surface is surface-treated by using a femtosecond laser, wherein

(i) the height of a to-be-processed surface of the first outer peripheral surface or the second outer peripheral surface is 0.001 mm to 10 mm greater than a surface thereof which has not been subjected to the treatment using the femtosecond laser, or
(ii) the depth of a to-be-processed surface of the first outer peripheral surface or the second outer peripheral surface is 0.001 mm to 10 mm greater than a surface thereof which has not been subjected to the treatment using the femtosecond laser.

**Patentansprüche**

**1.** Eine Implantatstruktur (100) umfassend eine Halterung (110), die als künstliche Zahnwurzel wirkt, wobei die Halterung Folgendes umfasst

einen zylindrischen Knochenkontaktabschnitt (111) mit einer ersten Außenumfangsfläche, an der ein Gewinde ausgebildet ist, und
einen gingivalen Kontaktabschnitt (112) mit einer zweiten Außenumfangsfläche, der an dem Knochenkontaktabschnitt angeordnet ist, und
wobei die erste Außenumfangsfläche eine Vielzahl von Nanovorsprüngen mit einer Breite von 10 nm bis 1000 nm und einem Seitenverhältnis von 1000:1 bis 1:1 beinhaltet, wodurch die Osseointegration gefördert wird, und die zweite Außenumfangsfläche eine Vielzahl von Nanovorsprüngen mit einer Breite von 10 nm bis 1000 nm und einem Seitenverhältnis von 1:1 bis 1:50 beinhaltet, wodurch eine antibakterielle Wirkung gefördert wird.

**2.** Die Implantatstruktur von Anspruch 1, wobei die Nanovorsprünge stumpfe Enden haben.

**3.** Die Implantatstruktur von Anspruch 1, wobei ein arithmetischer Mittelwert der Rauheit (Ra) der zweiten Außenumfangsfläche in einem Bereich von 1,0 $\mu$m bis 5,0 $\mu$m liegt.

**4.** Die Implantatstruktur von Anspruch 1, wobei ein arithmetischer Mittelwert der Rauheit (Ra) der ersten Außenumfangsfläche in einem Bereich von 0,5 $\mu$m bis 3,0 $\mu$m liegt.

**5.** Die Implantatstruktur von Anspruch 1, wobei die Implantatstruktur mindestens ein Material beinhaltet, das aus Titan oder einer Titanlegierung ausgewählt ist.

**6.** Die Implantatstruktur von Anspruch 1, wobei die Implantatstruktur mit einem Femtosekundenlaser oberflächenbehandelt ist.

**7.** Die Implantatstruktur von Anspruch 6, wobei die Oberflächenbehandlung darin besteht, dass die Oberfläche der ersten Außenumfangsfläche oder der zweiten Außenumfangsfläche, linear oder rasterförmig, ein- oder mehrmals mit einem Femtosekundenlaserstrahl mit einer Laserintensität von 1 W bis 5 W bestrahlt wird.

**8.** Die Implantatstruktur von Anspruch 7, wobei die erste Außenumfangsfläche oder die zweite Außenumfangsfläche unter Verwendung eines Femtosekundenlasers oberflächenbehandelt ist, wobei

(i) die Höhe einer zu bearbeitenden Oberfläche der ersten Außenumfangsfläche oder der zweiten Außenumfangsfläche 0,001 mm bis 10 mm größer ist als eine Oberfläche davon, die nicht der Behandlung mit dem Femtosekundenlaser unterzogen wurde, oder

(ii) die Tiefe einer zu bearbeitenden Oberfläche der ersten Außenumfangsfläche oder der zweiten Außenumfangsfläche 0,001 mm bis 10 mm größer ist als eine Oberfläche davon, die nicht der Behandlung mit dem Femtosekundenlaser unterzogen wurde.

**Revendications**

1. Une structure d'implant (100) comprenant un dispositif de fixation (110) qui agit comme une racine de dent artificielle, dans laquelle le dispositif de fixation comprend

   une partie de contact osseux cylindrique (111) ayant une première surface périphérique extérieure sur laquelle un filetage est formé et
   une partie de contact gingival (112) ayant une seconde surface périphérique extérieure et disposée sur la partie de contact osseux, et
   la première surface périphérique extérieure comprend une pluralité de nanoprotubérances ayant une largeur de 10 nm à 1000 nm et un rapport de forme de 1000:1 à 1:1, favorisant ainsi l'ostéointégration, et
   la seconde surface périphérique extérieure comprend une pluralité de nanoprotubérances ayant une largeur de 10 nm à 1000 nm et un rapport de forme de 1:1 à 1:50, favorisant ainsi l'activité antibactérienne.

2. La structure d'implant de la revendication 1, dans laquelle les nanoprotubérances ont des extrémités émoussées.

3. La structure d'implant de la revendication 1, dans laquelle une valeur de rugosité moyenne arithmétique (Ra) de la seconde surface périphérique extérieure est dans une plage de 1,0 μm à 5,0 μm.

4. La structure d'implant de la revendication 1, dans laquelle une valeur de rugosité moyenne arithmétique (Ra) de la première surface périphérique extérieure est dans une plage de 0,5 μm à 3,0 μm.

5. La structure d'implant de la revendication 1, dans laquelle la structure d'implant comprend au moins un matériau choisi parmi le titane ou un alliage de titane.

6. La structure d'implant de la revendication 1, dans laquelle la structure d'implant est traitée en surface avec un laser femtoseconde.

7. La structure d'implant de la revendication 6, dans laquelle le traitement de surface consiste à irradier avec un faisceau laser femtoseconde ayant une intensité laser de 1 W à 5 W sur la surface de la première surface périphérique extérieure ou de la seconde surface périphérique extérieure dans un type linéaire et un type de grille une ou plusieurs fois.

8. La structure d'implant de la revendication 7, dans laquelle la première surface périphérique extérieure ou la seconde surface périphérique extérieure est traitée en surface à l'aide d'un laser femtoseconde, dans laquelle

   (i) la hauteur d'une surface à traiter de la première surface périphérique extérieure ou de la seconde surface périphérique extérieure est supérieure de 0,001 mm à 10 mm à une surface de celle-ci qui n'a pas été soumise au traitement à l'aide du laser femtoseconde, ou
   (ii) la profondeur d'une surface à traiter de la première surface périphérique extérieure ou de la seconde surface périphérique extérieure est supérieure de 0,001 mm à 10 mm à une surface de celle-ci qui n'a pas été soumise au traitement à l'aide du laser femtoseconde.

# FIG. 1

# FIG. 2

# FIG. 3

# FIG. 4

# FIG. 5

200

- 210 LASER GENERATING UNIT
- 220 BEAM EXPANDING UNIT
- 230 BEAM ANGLE ADJUSTING UNIT
- 280 INHALATION UNIT
- 240 BEAM FOCUSING UNIT
- 260 CONTROL UNIT
- 270 OBJECT
- 250 ROTATING UNIT

EP 4 285 860 B1

# FIG. 6

# FIG. 7

(C)

(A)

(D)

(B)

(E)

(F)

FIG. 8

FIG. 9

EP 4 285 860 B1

# FIG. 10

# FIG. 11

(A) Line x1

(B) Line x10

(C) Line x100

(D) Grid x1

(E) Grid x10

(F) Grid x100

# FIG. 12

(A) Line x1

(B) Line x10

(C) Line x100

(D) Grid x1

(E) Grid x10

(F) Grid x100

# FIG. 13

(A)

(B)

(C)

# FIG. 14

(A)

(B)

(C)

(D)

Live (Green)/Dead (Red)

# FIG. 15

# FIG. 16

(A)

(B)

# FIG. 17

(A)

(B)

# FIG. 18

EP 4 285 860 B1

# FIG. 19

# FIG. 20

(A)

4h

(B)

24h

**REFERENCES CITED IN THE DESCRIPTION**

**Patent documents cited in the description**

- KR 2173456
- WO 2015033680 A